# EUROPEAN PATENT SPECIFICATION

(11) **EP 4 337 651 B1**
(45) Date of publication and mention of the grant of the patent: **04.02.2026**
(21) Application number: 22727785.2
(22) Date of filing: 03.05.2022
(51) Int. Cl.: C07D 401/12, C07D 405/12, C07D 409/12, A01N 43/713

(54) **SUBSTITUTED BENZAMIDES AS HERBICIDES**
SUBSTITUIERTE BENZAMIDE ALS HERBICIDE
BEZAMIDES SUBSTITUES COMME HERBICIDES

(30) Priority: 10.05.2021 IN 202111021131
(43) Date of publication of application: 20.03.2024
(73) Proprietor: Syngenta Crop Protection AG, 4058 Basel (CH)
(72) Inventor: BURTON, Paul Matthew, Bracknell, RG42 6EY (GB); TAYLOR, Nicholas John, Bracknell, RG42 6EY (GB); RAJAN, Ramya, Corlim Ilhas Goa 403 110 (IN); ARMSTRONG, Sarah, Bracknell, RG42 6EY (GB)
(74) Representative: SYNGENTA IP
(86) International application number: PCT/EP2022/061812
(87) International publication number: WO 2022/238178

(56) References cited:
- WO-A1-2012/028579
- WO-A1-2012/069366
- WO-A1-2020/079078

## Description

The present invention relates to novel herbicidal compounds, to processes for their preparation, to herbicidal compositions which comprise the novel compounds, and to their use for controlling weeds, in particular in crops of useful plants, or for inhibiting plant growth.

N-(tetrazol-5-yl)arylcarboxamides are disclosed in, for example, WO2012/028579. The present invention relates to novel arylcarboxamides, which exhibit surprisingly good control of weeds in rice crops.

Thus, according to the present invention there is provided a compound of Formula (I): 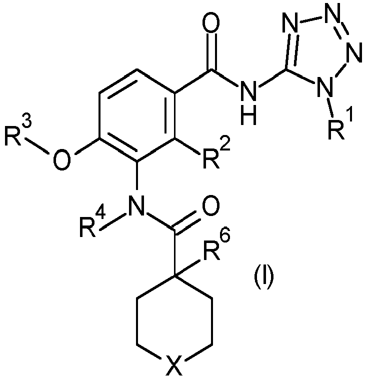
or an agronomically acceptable salt thereof,
wherein: -
X is selected from the group consisting of -O-, -S(O)ₚ- and -NR⁵;
R¹ is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
R³ is C₁-C₃haloalkyl;
R⁴ is selected from the group consisting of hydrogen, C₁-C₆alkyl-, C₁-C₆haloalkyl and C₃-C₆cycloalkyl;
R⁵ is selected from the group consisting of hydrogen, C₁-C₃alkyl- and C₁-C₃alkoxy-;
R⁶ is hydrogen or C₁-C₃alkyl; and
p is 0, 1 or 2.

C₁-C₆alkyl groups include, for example, methyl (Me, CH₃), ethyl (Et, C₂H₅), n-propyl *(n-*Pr), isopropyl (*i*-Pr), n-butyl (n-Bu), isobutyl (i-Bu), *sec-butyl* and tert-butyl (t-Bu).

C₃-C₆cycloalkyl- includes cyclopropyl (c-propyl (c-Pr)), cyclobutyl (c-butyl (c-Bu)), cyclopentyl (c-pentyl) and cyclohexyl (c-hexyl).

C₁-C₃alkoxy- includes, for example, methoxy- and ethoxy-.

Halogen (or halo) encompasses fluorine, chlorine, bromine or iodine. The same correspondingly applies to halogen in the context of other definitions, such as haloalkyl.

C₁-C₆haloalkyl includes, for example, fluoromethyl-, difluoromethyl-, trifluoromethyl-, chloromethyl-, dichloromethyl-, trichloromethyl-, 2,2,2-trifluoroethyl-, 2,2-difluoroethyl, 1,1-difluoroethyl, 1,1,2,2-tetrafluoroethyl, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl-, heptafluoro-n-propyl and perfluoro-n-hexyl. C₁-C₄haloalkyl includes, for example, fluoromethyl-, difluoromethyl-, trifluoromethyl-, chloromethyl-, dichloromethyl-, trichloromethyl-, 2,2,2-trifluoroethyl-, 2-fluoroethyl-, 2-chloroethyl-, pentafluoroethyl-, 1,1-difluoro-2,2,2-trichloroethyl-, 2,2,3,3-tetrafluoroethyl-, 2,2,2-trichloroethyl- and heptafluoro-n-propyl-.

C₁-C₄alkoxy-C₁-C₄alkyl- includes, for example, methoxyethyl-.

C₁-C₄haloalkoxy-C₁-C₄alkyl- includes, for example, trifluoromethoxyethyl-.

C₁-C₆alkyl-S- (alkylthio) is, for example, methylthio, ethylthio, propylthio, isopropylthio, n-butylthio, isobutylthio, sec-butylthio or tert-butylthio, preferably methylthio or ethylthio.

C₁-C₆alkyl-S(O)- (alkylsulfinyl) is, for example, methylsulfinyl, ethylsulfinyl, propylsulfinyl, isopropylsulfinyl, n-butylsulfinyl, isobutylsulfinyl, sec-butylsulfinyl or tert-butylsulfinyl, preferably methylsulfinyl or ethylsulfinyl.

C₁-C₆alkyl-S(O)₂- (alkylsulfonyl) is, for example, methylsulfonyl, ethylsulfonyl, propylsulfonyl, isopropylsulfonyl, n-butylsulfonyl, isobutylsulfonyl, sec-butylsulfonyl or tert-butylsulfonyl, preferably methylsulfonyl or ethylsulfonyl.

In one embodiment of the present invention X is -O- or -S(O)ₚ- (e.g -S-, -S(O) or -S(O)₂-).

In one embodiment of the present invention, X is -O-.

In another embodiment of the present invention, X is -S(O)ₚ- (e.g -S-, -S(O) or -S(O)₂-).

In another embodiment of the present invention, X is -NR⁵-, wherein R⁵ is selected from the group consisting of hydrogen, C₁-C₃alkyl- and C₁-C₃alkoxy- (e.g methoxy-).

In a preferred embodiment of the present invention, R¹ is selected from the group consisting of methyl, ethyl and n-propyl, preferably methyl.

In a preferred embodiment of the present invention, R² is selected from the group consisting of methyl, Cl, -CF₃ and -SO₂methyl, more preferably Cl.

In another preferred embodiment of the present invention, R³ is -CF₃ or -CHF₂.

In one embodiment of the present invention, R⁴ hydrogen or C₁-C₄alkyl- (preferably methyl), preferably hydrogen.

In another embodiment of the present invention R⁶ is hydrogen or methyl, preferably hydrogen.

Compounds of Formula (I) (and certain intermediate compounds used to synthesise compound of Formula (I)) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

The present invention also includes all possible geometric and tautomeric forms of a compound of formula (I).

The present invention also includes agronomically acceptable salts that the compounds of Formula (I) may form with amines (for example ammonia, dimethylamine and triethylamine), alkali metal and alkaline earth metal bases or quaternary ammonium bases. Among the alkali metal and alkaline earth metal hydroxides, oxides, alkoxides and hydrogen carbonates and carbonates used as salt formers, emphasis is to be given to the hydroxides, alkoxides, oxides and carbonates of lithium, sodium, potassium, magnesium and calcium, but especially those of sodium, magnesium and calcium. The corresponding trimethylsulfonium salt may also be used.

The compounds of Formula (I) according to the invention can be used as herbicides by themselves, but they are generally formulated into herbicidal compositions using formulation adjuvants, such as carriers, solvents and surface-active agents (SFAs). Thus, the present invention further provides a herbicidal composition comprising a herbicidal compound of the present invention and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-isopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

The compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, benquitrione, bensulfuron (including bensulfuron-methyl), bentazone, bicyclopyrone, bilanafos, bipyrazone, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, dioxopyritrione, diquat dibromide, diuron, epyrifenacil, ethalfluralin, ethofumesate, fenoxaprop (including fenoxaprop-P-ethyl), fenoxasulfone, fenpyrazone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, flupyrsulfuron (including flupyrsulfuron-methyl-sodium), fluroxypyr (including fluroxypyr-meptyl), fomesafen, foramsulfuron, glufosinate (including L-glufosinate and the ammonium salts of both), glyphosate (including the diammonium, isopropylammonium and potassium salts thereof), halauxifen (including halauxifen-methyl), haloxyfop (including haloxyfop-methyl), hexazinone, hydantocidin, imazamox (including R-imazamox), imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimisoxafen, rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tripyrasulfone, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester, 4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate, prop-2-ynyl 4-amino-3-chloro-5-fluoro-6-(7-fluoro-1H-indol-6-yl)pyridine-2-carboxylate and cyanomethyl 4-amino-3-chloro-5-fluoro-6-(7-fluoro-1H-indol-6-yl)pyridine-2-carboxylate), 3-ethylsulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonylmethyl)-N-(5-methyl-1,3,4-oxadiazol-2-yl)-5-(trifluoromethyl)-[1,2,4]triazolo[4,3-a]pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide, ethyl 2-[[3-[[3-chloro-5-fluoro-6-[3-methyl-2,6-dioxo-4-(trifluoromethyl)pyrimidin-1-yl]-2-pyridyl]oxy]acetate and 6-chloro-4-(2,7-dimethyl-1-naphthyl)-5-hydroxy-2-methyl-pyridazin-3-one.

The mixing partners of the compound of Formula I may also be in the form of esters or salts, as mentioned e.g. in The Pesticide Manual, Sixteenth Edition, British Crop Protection Council, 2012.

The compound of Formula I can also be used in mixtures with other agrochemicals such as fungicides, nematicides or insecticides, examples of which are given in The Pesticide Manual.

The mixing ratio of the compound of Formula I to the mixing partner is preferably from 1: 100 to 1000: 1.

The mixtures can advantageously be used in the above-mentioned formulations (in which case "active ingredient" relates to the respective mixture of compound of Formula I with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyr-diethyl), metcamifen and oxabetrinil. Particularly preferred are mixtures of a compound of Formula I with cyprosulfamide, isoxadifen-ethyl, cloquintocet-mexyl and/or metcamifen.

The safeners of the compound of Formula I may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of Formula I to safener is from 100:1 to 1:10, especially from 20:1 to 1:1.

The mixtures can advantageously be used in the above-mentioned formulations (in which case "active ingredient" relates to the respective mixture of compound of Formula I with the safener).

The present invention still further provides a method of controlling weeds at a locus said method comprising application to the locus of a weed controlling amount of a composition comprising a compound of Formula (I). Moreover, the present invention further provides a method of selectively controlling weeds at a locus comprising crop plants and weeds, wherein the method comprises application to the locus of a weed controlling amount of a composition according to the present invention. 'Controlling' means killing, reducing or retarding growth or preventing or reducing germination. Generally, the plants to be controlled are unwanted plants (weeds). 'Locus' means the area in which the plants are growing or will grow. Some crop plants may be inherently tolerant to herbicidal effects of compounds of Formula (I). However, in some instances tolerance may need to be engineered into the crop plant, for example by way of genetic engineering. Thus, it is possible that the crop plant is rendered tolerant to HPPD-inhibitors via genetic engineering. Methods of rending crop plants tolerant to HPPD-inhibitors are known, for example from WO0246387. Thus in an even more preferred embodiment the crop plant is transgenic in respect of a polynucleotide comprising a DNA sequence which encodes an HPPD-inhibitor resistant HPPD enzyme derived from a bacterium, more particularly from *Pseudomonas fluorescens* or *Shewanella colwelliana,* or from a plant, more particularly, derived from a monocot plant or, yet more particularly, from a barley, maize, wheat, rice, *Brachiaria, Cenchrus, Lolium, Festuca, Setaria, Eleusine, Sorghum or Avena* species. Several HPPD-tolerant soybean transgenic "events" are known and include for example SYHT04R (WO2012/082542), SYHT0H2 (WO2012/082548) and FG72. Other polynucleotide sequences that can be used to provide plants which are tolerant to the compounds of the present invention are disclosed in, for example, WO2010/085705 and WO2011/068567. Crop plants in which the composition according to the invention can be used thus include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

The rates of application of compounds of Formula I may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula I according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Crop plants are to be understood as also including those crop plants which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield^{®} summer rape (canola). Examples of crops that have been rendered tolerant to herbicides by genetic engineering methods include e.g. glyphosate- and glufosinate-resistant maize varieties commercially available under the trade names RoundupReady^{®} and LibertyLink^{®}.

Crop plants are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK^{®} (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut^{®} (maize), Yield Gard^{®} (maize), NuCOTIN33B^{®} (cotton), Bollgard^{®} (cotton), NewLeaf^{®} (potatoes), NatureGard^{®} and Protexcta^{®}. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

The compositions can be used to control unwanted plants (collectively, 'weeds'). The weeds to be controlled may be both monocotyledonous species, for example Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum, and dicotyledonous species, for example Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium. Weeds can also include plants which may be considered crop plants but which are growing outside a crop area ('escapes'), or which grow from seed left over from a previous planting of a different crop ('volunteers'). Such volunteers or escapes may be tolerant to certain other herbicides.

The compounds of the present invention can be prepared according to the following schemes.

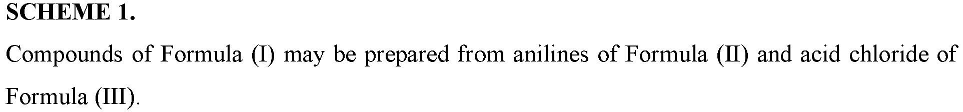

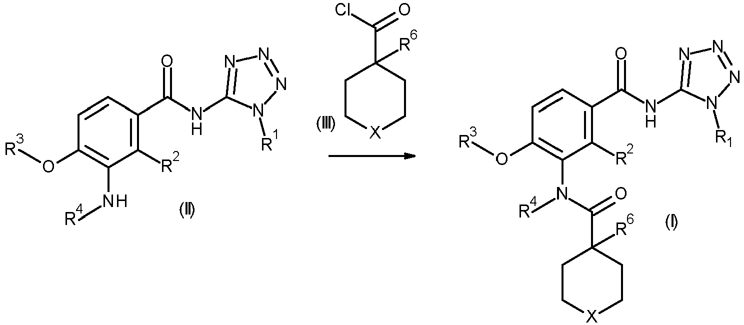

The compound of Formula (II) is treated with the compound of Formula (III) in a suitable solvent, for example acetonitrile, to give the compound of Formula (I).

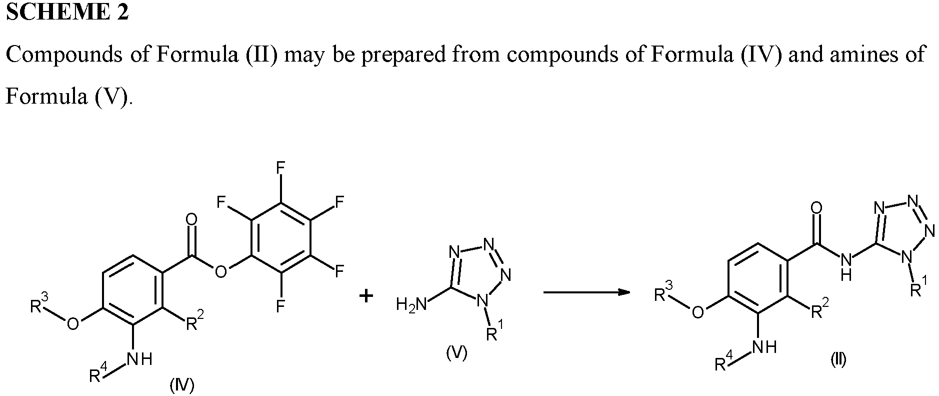

The compound of Formula (IV) and compound of Formula (V) are treated with a base, for example 2-tert-butylimino-2-diethylamino-1,3-dimethylperhydro-1,3,2-diazaphosphorine, in a suitable solvent, for example acetonitrile, to give the compound of Formula (II).

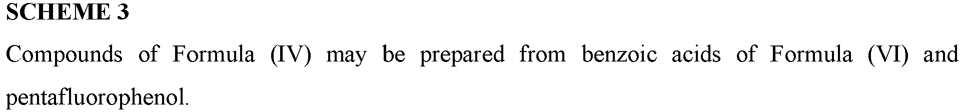

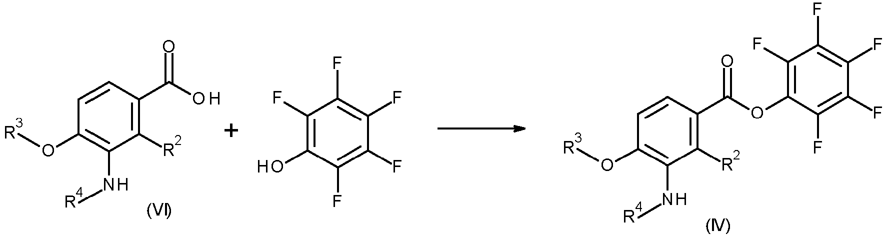

The compound of Formula (VI) is treated with pentafluorophenol and an ester coupling reagent, for example 1-ethyl-3-(3-dimethylaminopropyl)carbodiimide, in a suitable solvent, for example acetonitrile, to give the compound of Formula (VI).

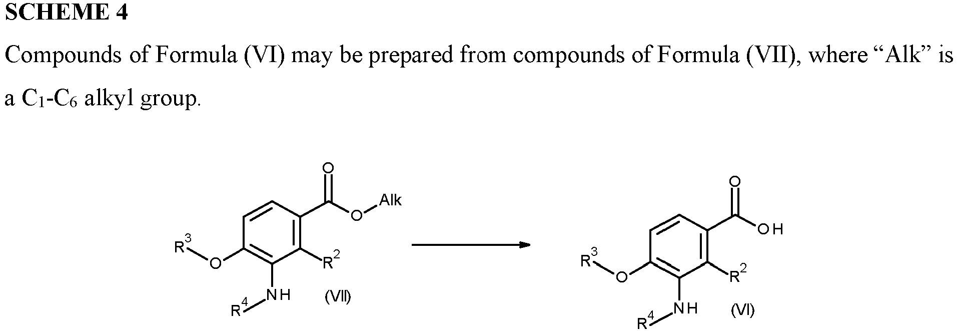

The compound of Formula (VII) is treated with a hydroxide base, for example lithium hydroxide, in a suitable solvent, for example a 4:1 mixture of tetrahydrofuran : water, to give the compound of Formula (VI).

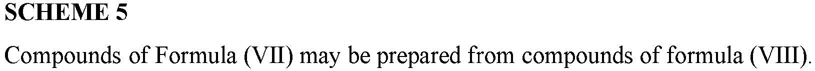

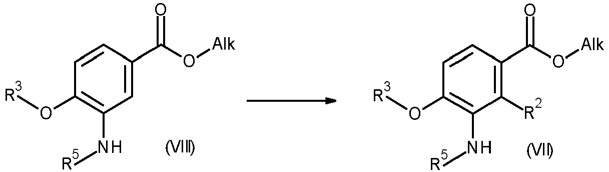

The conditions used in this transformation will depend on the nature of R². For example, where R² is chlorine, the compound of Formula (VIII) is treated with sulfuryl chloride and a catalytic amount of diisopropylamine.

Alternatively, compounds of Formula (VII) may be prepared from compounds of Formula (VII), where R² = chlorine. For example, a compound of Formula (VII) where R² = chlorine may be treated with [1,3-bis(2,6-diisopropylphenyl)imidazol-2-ylidene](3-chloropyridyl)palladium(II) dichloride, trimethyl boroxine and potassium carbonate, in a suitable solvent, for example 1,4-dioxane, to give the corresponding compound of Formula (VII) where R² = methyl.

The following non-limiting examples provide specific synthesis methods for representative compounds of the present invention, as referred to in Table 1 provided herein.

### Example 1: Preparation of Compound 1.001

### Step 1:

To a round bottom flask containing methyl 3-amino-4-(trifluoromethoxy)benzoate (3.00 g, 12.8 mmol) was added toluene (45 mL) and diisopropylamine (0.13 g, 1.3 mmol). The reaction mixture was heated to 70 °C. Sulfuryl chloride (1.55 g, 11.5 mmol) was added to the reaction mixture slowly over 10 min, and the reaction mixture quickly turned yellow and gas was released. After the addition was complete, the reaction mixture was stirred for an additional 40 min, then allowed to cool. Water (30 mL) was added and toluene was removed under reduced pressure. The reaction mixture was added to a separating funnel and extracted with ethyl acetate (2 x 60 mL). The combined organic layers were washed with brine (30 mL), dried over MgSO₄ and concentrated. The residue was purified via reverse phase column chromatography (acetonitrile+0.1% formic acid/water+0.1% formic acid gradient 40:60 to 80:20) to afford methyl 3-amino-2-chloro-4-**(trifluoromethoxy)benzoate** (1.88 g, 6.97 mmol, 55%) as a yellow oil. ¹H NMR (400 MHz, CD₃OD): δ = 7.19 (dq, 1H), 7.07 (d, 1H), 3.89 (s, 3H).

### Step 2:

To a stirred solution of methyl 3-amino-2-chloro-4-(trifluoromethoxy)benzoate (10.0 g, 37.1 mmol) in tetrahydrofuran (120 mL) and water (30 mL) at room temperature was added lithium hydroxide monohydrate (4.67 g, 111 mmol). The mixture was stirred at room temperature for 22 hours. The mixture was then cooled to 0°C (ice-water bath). The reaction was quenched by slow addition of 2 M aq. HCl (100 mL). The cooling bath was removed and the mixture was allowed to warm to room temperature. EtOAc (100 mL) and brine (50 mL) were added and the phases were separated. The aqueous phase was extracted with EtOAc (100 mL). The combined organic phases were dried (MgSO₄), filtered and concentrated in vacuo to afford 3-amino-2-chloro-4-(trifluoromethoxy)benzoic acid (9.25 g, 34.4 mmol, 93%) as a beige solid which was used in the next step without further purification. ¹H NMR (400 MHz, CD₃OD): δ = 7.20 - 7.15 (m, 1H), 7.13 - 7.08 (m, 1H).

### Step 3:

To a stirred suspension of 3-amino-2-chloro-4-(trifluoromethoxy)benzoic acid (9.24 g, 36.2 mmol) and 2,3,4,5,6-pentafluorophenol (7.32 g, 39.8 mmol) in dichloromethane (139 mL) at room temperature was added 3-(ethyliminomethyleneamino)-N,N-dimethyl-propan-1-amine hydrochloride (8.32 g, 43.4 mmol). The mixture was stirred at room temperature for one hour. The reaction was then quenched by addition of sat. aq. NaHCO₃ (100 mL). The mixture was stirred at room temperature for a further 5 minutes. Dichloromethane (40 mL) was added and the phases were separated. The aqueous phase was extracted with dichloromethane (50 mL). The combined organic phases were passed through a phase separator cartridge. The filtrate was adsorbed onto silica and the crude product was purified by flash column chromatography (cyclohexane/EtOAc gradient 98:2 to 93:7). Product-containing fractions were combined and concentrated *in vacuo* to afford (2,3,4,5,6-pentafluorophenyl) 3-amino-2-chloro-4-(trifluoromethoxy)benzoate (12.54 g, 29.74 mmol, 82%) as a pale yellow crystalline solid. ¹H NMR (400 MHz, CDCl₃) δ = 7.53 (d, 1H), 7.26 - 7.22 (m, 1H), 4.62 (br s, 2H).

### Step 4:

To a stirred solution of (2,3,4,5,6-pentafluorophenyl) 3-amino-2-chloro-4-(trifluoromethoxy)benzoate (6.00 g, 14.2 mmol) in acetonitrile (120 mL) at room temperature was added 1-ethyltetrazol-5-amine (3.54 g, 31.3 mmol) followed by 2-tert-butylimino-N,N-diethyl-1,3-dimethyl-1,3,2λ⁵-diazaphosphinan-2-amine (9.34 mL, 31.3 mmol). The mixture was stirred at room temperature for the 30 minutes. Then reaction was then quenched by addition of 2 M aq. HCl (100 mL). The mixture was stirred at room temperature for a further 5 minutes, then diluted with EtOAc (100 mL). The phases were separated. The aqueous phase was extracted with EtOAc (100 mL). The combined organic phases were washed with brine (100 mL), dried (MgSO₄), filtered and adsorbed onto C18-silica. The crude product was partially purified by reverse phase chromatography (acetonitrile+0.1% formic acid/water+0.1% formic acid gradient 40:60 to 60:40). Product-containing fractions were combined, partially concentrated *in vacuo* to remove acetonitrile and lyophilised to afford partially purified material. The partially purified material was adsorbed onto C18-silica and further purified by reverse phase chromatography (acetonitrile+0.1% formic acid/water+0.1% formic acid gradient 35:65 to 55:45). Clean fractions were combined and partially concentrated *in vacuo* to remove MeCN. The mixture was then diluted with brine (50 mL) and extracted with EtOAc (2 x 150 mL). The combined organic phases were dried (MgSO₄), filtered and concentrated in vacuo to afford 3-amino-2-chloro-N-(1-ethyltetrazol-5-yl)-4-(trifluoromethoxy)benzamide (4.26 g, 11.5 mmol, 81%) as a white foam. ¹H NMR (400 MHz, CD₃OD): δ = 7.27 (dq, 1H), 6.94 (d, 1H), 4.42 (q, 2H), 1.58 (t, 3H).

### Step 5:

Tetrahydropyran-4-carboxylic acid (1.10 g) was weighed out into a robot tube and charged with a stirrer bar. These were then dissolved in DCM (4 mL) and 2 drops of DMF were added. Oxalyl Chloride (0.72 mL) was added and the reaction was stirred at RT for 2 h. Half the material (2 mL) was transferred to another robot tube. 3-amino-2-chloro-N-(1-ethyltetrazol-5-yl)-4-(trifluoromethoxy)benzamide (279 mg, 0.83 mmol) in acetonitrile (2 mL) was added to the new tube and it was heated at 50°C for 18 h. MeOH (2 mL) was added to react with any remaining acid chloride. The solvent was removed using a Genevac. Purification via reverse phase prep. HPLC gave N-[2-chloro-3-[(1-methyltetrazol-5-yl)carbamoyl]-6-(trifluoromethoxy)phenyl]-tetrahydropyran-4-carboxamide (260 mg, 0.579 mmol, 70%) as a solid. ¹H NMR (400 MHz, DMSO-d₆): 11.93 (br s, 1H), 9.94 (s, 1H), 7.81 (d, *J =* 8.6 Hz, 1H), 7.62 (d, *J =* 8.8 Hz, 1H), 4.00 (s, 3H), 3.94 - 3.87 (m, 2H), 3.43 - 3.36 (m, 2H), 2.77 - 2.65 (m, 1H), 1.79 - 1.62 (m, 4H)

### Example 2: Preparation of Compound 1.002.

### Step 1

To a flask containing methyl 4-hydroxy-3-nitro-benzoate (5.2 g, 26 mmol) was added N,N-dimethylformamide (52 mL), potassium carbonate (4.6 g, 32 mmol) and sodium 2-chloro-2,2-difluoroacetate (4.9 g, 32 mmol). The reaction changed to brown in colour and was heated to 75 °C and stirred for 18 h. The reaction mixture quenched with 2 N HCl and extracted with ethylacetate. The organic layer was dried with anhydrous sodium sulphate, concentrated and purified by flash chromatography (10% EtOAc / cyclohexane) to give methyl 4-(difluoromethoxy)-3-nitrobenzoate (2.4 g, 9.9 mmol, 38%) as a yellow gum. ¹H NMR (d6-DMSO): 8.58-7.20 (4H, m), 3.91 (3H, s).

### Step 2

Methyl 4-(difluoromethoxy)-3-nitro-benzoate (16 g, 45 mmol) was dissolved in ethanol (160 mL) followed by ammonium chloride (14.5 g, 272 mmol), iron power (8.0 g, 142 mmol) and water (160 mL) The reaction mass was heated at reflux at 95 °C for 18 h. The reaction was filtered through celite and partitioned between EtOAc and brine. The organic layer was separated and dried over anhydrous Na2SO4 and concentrated. Flash chromatography (5-20% EtOAc : Cyclohexane) gave methyl 3-amino-4-(difluoromethoxy)benzoate (8.5 g, 37 mmol, 82%) as and off white solid. ¹H NMR (d6-DMSO): 7.42-6.99 (4H, m), 5.39 (2H, s), 3.81 (3H, s).

### Step 3

A three neck RBF was charged with methyl 3-amino-4-(difluoromethoxy)benzoate (15 g, 69. Mmol) and diisopropylammonium chloride (1.43 g,10.4 mmol). Toluene (150 mL) was added and the reaction mixture was heated to 60 °C. 1,3-dichloro-5,5-dimethyl-imidazolidine-2,4-dione (8.17, 41.4 mmol) in 1,4-dioxane (45 ml) was added drop wise in over 5 min. After the addition was complete the reaction was cooled to RT and was quenched with saturated sodium meta bisulphite. After stirring for 5 min it was extracted with ethyl acetate. The organic layer was dried over anhydrous sodium sulphate, concentrated and purified by chromatography (0-10% ethylacetate: cyclohexane) to give methyl 3-amino-2-chloro-4-(difluoromethoxy)benzoate (11.5 g, 43.4 mmol, 63%) as an off-white solid. ¹H NMR (CDCl₃): 7.24 (1H, d), 7.02 (1H, d), 6.56 (1H, t), 4.49 (2H, brs), 3.94 (3H, s).

### Step-4

To a stirred solution of methyl 3-amino-2-chloro-4-(difluoromethoxy)benzoate (4.45 g, 17.7 mmol) in methanol (50 mL), lithium hydroxide (1.69 g, 70.7 mmol) dissolved in water (12.4 mL) was added. The reaction mixture was stirred at ambient temperature for 12 h. After completion, the mixture was acidified (till acidic to pH paper) by drop wise addition of aqueous citric acid solution. The resulting aqueous solution was extracted with ethyl acetate (3X 50 mL). Combined organic layers were washed with brine, dried over sodium sulphate and concentrated to afford 3-amino-2-chloro-4-(difluoromethoxy) benzoic acid (4.0 g, 85.7% Yield) as yellow solid. ¹H NMR (400 MHz, d6-DMSO): 13.3 (br s, 1 H) 7.18 (t, 1H) 7.10 (d, 1 H) 6.99 (d, 1 H) 5.49 (s, 2H)

### Step 5

To a stirred solution of 3-amino-2-chloro-4-(difluoromethoxy)benzoic acid (800 mg, 3.37 mmol) in benzotrifluoride (10 mL) and acetonitrile (5 mL) were added 2,3,4,5,6-pentafluorophenol (680 mg, 3.70 mmol), 3-(ethyliminomethyleneamino)-N,N-dimethyl-propan-1-amine hydrochloride (626 mg, 4.04 mmol) and N,N-dimethylpyridin-4-amine (82 mg, 0.673 mmol) and the reaction was stirred at ambient temperature under a nitrogen atmosphere for 12 hrs. The reaction mixture was concentrated under vacuum and directly purified by flash chromatography using 10% ethyl acetate in cyclohexane as eluent to afford (2,3,4,5,6-pentafluorophenyl) 3-amino-2-chloro-4-(difluoromethoxy)benzoate (1.2 g, 88% Yield) as a white solid. ¹H NMR (400 MHz, CDCl₃): 7.56 (d,1 H) 7.12 (d,1 H) 6.64 (t, 1 H) 4.61 (br s, 2 H)

### Step 6

To a stirred solution of 1-methyltetrazol-5-amine (1.080 g, 3.5 mmol) in DMF (10 mL), 2-tert-butylimino-2-diethylamino-1,3-dimethylperhydro-1,3,2-diazaphosphorine (2.2 mL, 7.1 mmol) was added followed by (2,3,4,5,6-pentafluorophenyl) 3-amino-2-chloro-4-(difluoromethoxy)benzoate (1.3 g, 3.2 mmol) at ambient temperature and stirred for 2h. The reaction mass was diluted with ethyl acetate (25 mL) and acidified with 1N HCl (until acidic to pH paper). The combined organic layers were washed with brine, dried over sodium sulphate and concentrated to afford 3-amino-2-chloro-4-(difluoromethoxy)-N-(1-methyltetrazol-5-yl)benzamide (1.08 g, 80% pure, 84 % yield) as a solid, which was used for next step without further purifications.

### Step 7

To a stirred solution of 3-amino-2-chloro-4-(difluoromethoxy)-N-(1-methyltetrazol-5-yl)benzamide (0.200 g, 0.502 mmol) in acetonitrile (4 mL) was added tetrahydropyran-4-carbonyl chloride (0.223 g, 1.51 mmol) and the reaction mass was stirred at 60 °C for 2 hrs. The reaction mixture was concentrated under vacuum to give crude mass, which was purified by reversed phase column chromatography using acetonitrile: acetonitrile / water (0.1% formic acid) as eluent to afford as N-[2-chloro-6-(difluoromethoxy)-3-[(1-methyltetrazol-5-yl)carbamoyl] phenyl]tetrahydropyran-4-carboxamide (89 mg, 41% Yield) as a white solid. ¹H NMR (400 MHz, d6-DMSO): 1.62 - 1.79 (m, 4 H) 2.65 - 2.77 (m, 1 H) 3.25 - 3.40 (m, 2 H) 3.89-3.91 (m, 2 H) 4.00 (s, 3 H) 7.21 (t, 2 H) 7.40 (d, 1 H) 7.76 (d, 1H) 9.76 (br s, 1 H) 11.87 (br s, 1 H).

**TABLE 1 - Examples of herbicidal compounds of the present invention.**

| **Compound Number** | **Structure** | **¹H NMR (in d6-DMSO unless otherwise indicated)** |
|---|---|---|
| 1.001 | 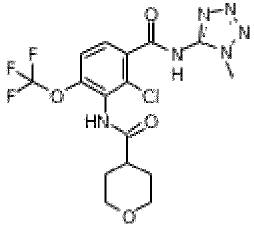 | 11.93 (br s, 1H), 9.94 (s, 1H), 7.81 (d, J = 8.6 Hz, 1H), 7.62 (d, J = 8.8 Hz, 1H), 4.00 (s, 3H), 3.94 - 3.87 (m, 2H), 3.43 - 3.36 (m, 2H), 2.77 - 2.65 (m, 1H), 1.79 - 1.62 (m, 4H) |
| 1.002 | 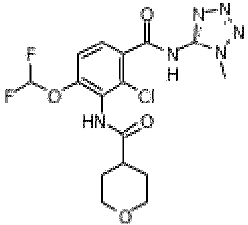 | 1.62 - 1.79 (m, 4 H) 2.65 - 2.77 (m, 1 H) 3.25 - 3.40 (m, 2 H) 3.89-3.91 (m, 2 H) 4.00 (s, 3 H) 7.21 (t, 2 H) 7.40 (d, 1 H) 7.76 (d, 1H) 9.76 (br s, 1 H) 11.87 (br s, 1 H). |
| 1.003 | 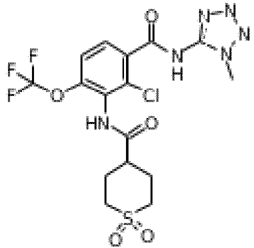 | 11.91 (s, 1H), 10.14 (s, 1H), 7.83 (d, 1H), 7.63 (d, 1H), 4.00 (s, 3H), 3.32 - 3.21 (m, 2H), 3.20 - 3.12 (m, 2H), 2.84 (tt, 1H), 2.28 - 2.18 (m, 2H), 2.18 - 2.07 (m, 2H). |
| 1.004 | 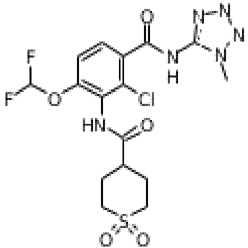 | 11.84 (s, 1H), 9.94 (s, 1H), 7.77 (d, 1H), 7.40 (d, 1H), 7.22 (t, 1H), 3.99 (s, 3H), 3.29 - 3.20 (m, 2H), 3.20 - 3.12 (m, 2H), 2.87 - 2.79 (m, 1H), 2.29 - 2.19 (m, 2H), 2.18 - 2.08 (m, 2H) |
| 1.005 | 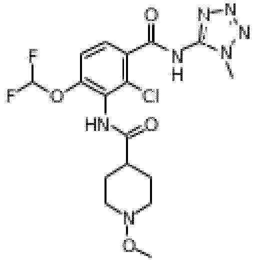 | |
| 1.006 | 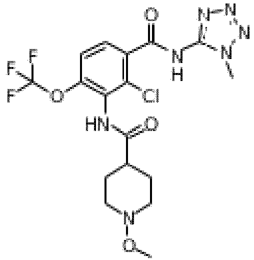 | |
| 1.007 | 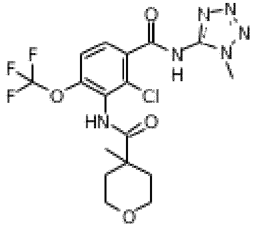 | 11.93 (s, 1H), 9.59 (s, 1H), 7.83 (d, 1H), 7.63 (d, 1H), 4.00 (s, 3H), 3.72 (td, 2H), 3.61 - 3.41 (m, 2H + overlaps with water peak), 2.12 (br d, 2H), 1.50 (ddd, 2H), 1.30 (s, 3H). |
| 1.008 | 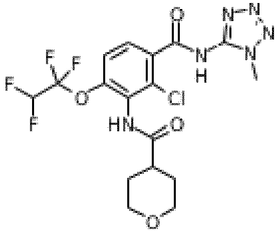 | 7.74 (d, 1H), 7.55 (d, 1H), 6.37 (br t, 1H), 4.07 (s, 3H), 4.05 - 3.98 (m, 2H), 3.58 - 3.47 (m, 2H), 2.84 - 2.70 (m, 1H), 1.93 - 1.78 (m, 4H). |
| 1.009 | 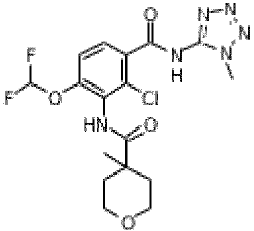 | 11.85 (s, 1H), 9.41 (s, 1H), 7.77 (d, 1H), 7.39 (d, 1H), 7.23 (t, 1H), 4.00 (s, 3H), 3.71 (dt, 2H), 3.55 - 3.45 (m, 2H), 2.12 (br d, 2H), 1.48 (ddd, 2H), 1.29 (s, 3H). |
| 1.010 | 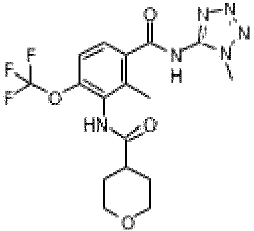 | 11.67 (s, 1H), 9.62 (s, 1H), 7.69 (d, 1H), 7.42 (d, 1H), 3.99 (s, 3H), 3.95 - 3.87 (m, 2H), 3.39 (td, 2H), 2.75 - 2.65 (m, 1H), 2.27 (s, 3H), 1.79 - 1.62 (m, 4H). |
| 1.011 | 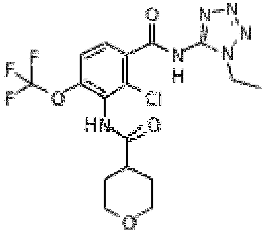 | 11.83 (s, 1H), 9.93 (s, 1H), 7.80 (d, 1H), 7.65 - 7.59 (m, 1H), 4.36 (q, 2H), 3.96 - 3.86 (m, 2H), 3.39 (td, 2H), 2.77 - 2.65 (m, 1H), 1.79 - 1.61 (m, 4H), 1.46 (t, 3H) |
| 1.012 | 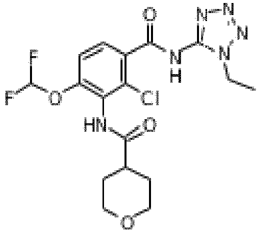 | 1H NMR (400 MHz, DMSO-d6) δ ppm 11.75 (br s, 1H), 9.74 (s, 1H), 7.37 (d, 1H), 6.97 - 7.39 (m, 2H), 4.35 (q, 2H), 3.90 (br d, 2H), 3.35 - 3.43 (m, 2H), 2.68 - 2.74 (m, 1H), 1.60 - 1.80 (m, 4H), 1.46 (t, 3H) |
| 1.013 | 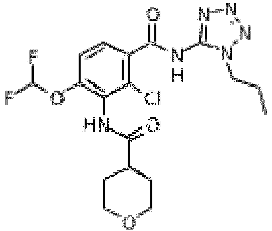 | |
| 1.014 | 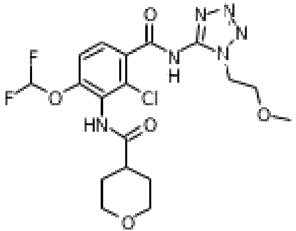 | |
| 1.015 | 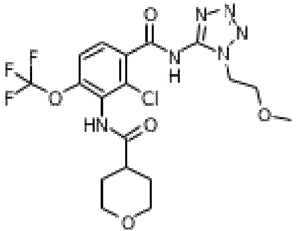 | |
| 1.016 | 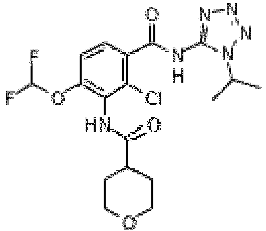 | |
| 1.017 | 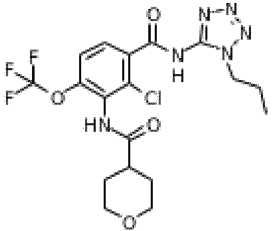 | |
| 1.018 | 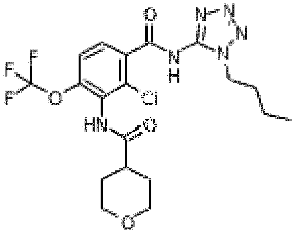 | |
| 1.019 | 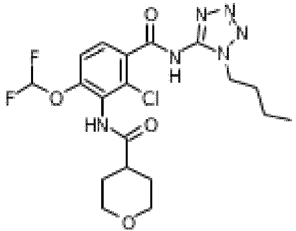 | |
| 1.020 | 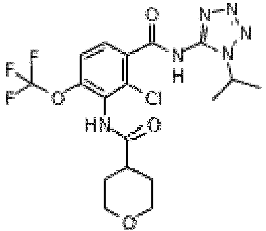 | |
| 1.021 | 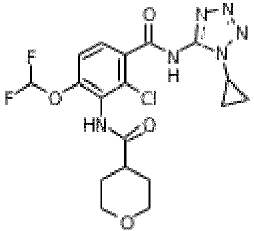 | |
| 1.022 | 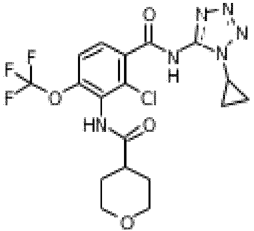 | |
| 1.023 | 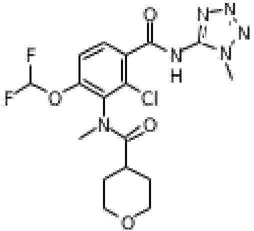 | |
| 1.024 | 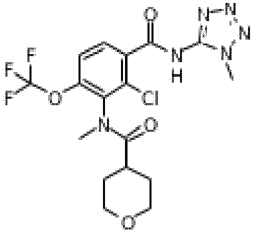 | |
| 1.025 | 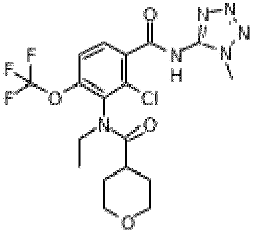 | |
| 1.026 | 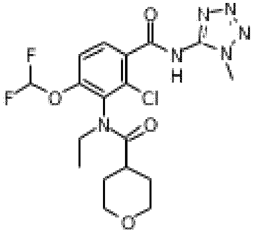 | |

### Biological Examples

Seeds of test species were sown in standard soil in pots. After cultivation for one day under controlled conditions in a glasshouse (at 24/16°C, day/night; 14 hours light; 65 % humidity), the plants were sprayed with an aqueous spray solution derived from the formulation of the technical active ingredient in 0.6 ml acetone and 45 ml formulation solution containing 10.6% Emulsogen EL (Registry number 61791-12-6), 42.2% N-methyl pyrrolidone, 42.2% dipropylene glycol monomethyl ether (CAS RN 34590-94-8) and 0.2 % X-77 (CAS RN 11097-66-8).

The test plants were then grown in a glasshouse under controlled conditions in a glasshouse (at 24/16°C, day/night; 14 hours light; 65 % humidity) and watered twice daily. After 14 days, the test was evaluated (100 = total damage to plant; 0 = no damage to plant).

Test species: *Oryza sativa* (ORYSA, Rice), *Euphorbia heterophylla* (EPHHL), *Sinapis arvensis* (SINAR), *Panicum miliaceum* (PANMI).

**TABLE B1**

| **Compound** | POST Application | | | | |
|---|---|---|---|---|---|
| C1 | 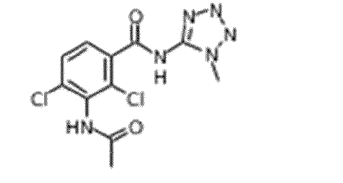 | | | | |
| | Rate g/ha | *ORYSA* | *EPHHL* | *SINAR* | *PANMI* |
| | 250 | 90 | 100 | 100 | 90 |
| | 130 | 80 | 100 | 80 | 80 |
| | 30 | 60 | 90 | 70 | 20 |
| | | | | | |
| 1.001 | 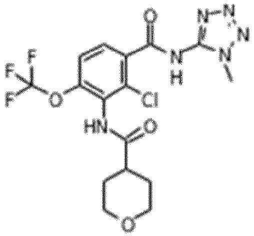 | | | | |
| | Rate g/ha | *ORYSA* | *EPHHL* | *SINAR* | *PANMI* |
| | 250 | 0 | 100 | 100 | 100 |
| | 130 | 0 | 100 | 100 | 100 |
| | 30 | 0 | 100 | 100 | 90 |
| 1.002 | 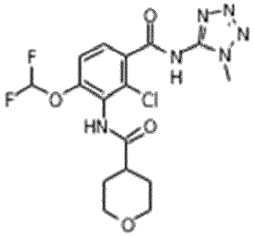 | | | | |
| | Rate g/ha | *ORYSA* | *EPHHL* | *SINAR* | *PANMI* |
| | 250 | 20 | 100 | 100 | 100 |
| | 130 | 10 | 100 | 100 | 100 |
| | 30 | 0 | 90 | 90 | 90 |

C1 is compound 4-659 disclosed in WO2012/028579. The experimental data shows that the compounds of the present invention provide significantly less damage to the rice plants *(ORYSA)* compared to C1, but still retain a good level of weed control.

**TABLE B2**

| Seeds of a variety of test species are sown in standard soil in pots *Amaranthus retoflexus* (AMARE), *Setaria faberi* (SETFA), *Echinochloa crus-galli* (ECHCG), *Ipomoea hederacea* (IPOHE)). After cultivation for one day (pre-emergence) or after 8 days cultivation (post-emergence) under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 130 g/h unless otherwise indicated. The test plants are then grown in a glasshouse under controlled conditions in a glasshouse (at 24/16°C, day/night; 14 hours light; 65 % humidity) and watered twice daily. After 13 days for pre and post-emergence, the test is evaluated for the percentage damage caused to the plant. The biological activities are shown in the following table on a five-point scale (5 = 80-100%; 4 = 60-79%; 3=40-59%; 2=20-39%; 1=0-19%). | | | | | | | | |
|---|---|---|---|---|---|---|---|---|
| | PRE Application | | | | POST Application | | | |
| **Compound** | AMARE | IPOHE | ECHCG | SETFA | AMARE | IPOHE | ECHCG | SETFA |
| 1.001 | 5 | 4 | 5 | 5 | 5 | 5 | 5 | 5 |
| 1.002 | 5 | 5 | 5 | 5 | 5 | 4 | 5 | 5 |
| 1.007 | 5 | 3 | 2 | 1 | 5 | - | 5 | 5 |
| 1.009 | 5 | 2 | 1 | 1 | 5 | 4 | 4 | 2 |
| 1.010 | 5 | 2 | 3 | 2 | 4 | 4 | 5 | 5 |
| 1.011 | 5 | 5 | 5 | 5 | 5 | 5 | 5 | 5 |
| 1.012 | 4 | 4 | 5 | 3 | 5 | 4 | 5 | 5 |

| | | | | | | | | |
|---|---|---|---|---|---|---|---|---|
| "-" = Data not captured. | | | | | | | | |

## Claims

1. A compound of Formula (I): 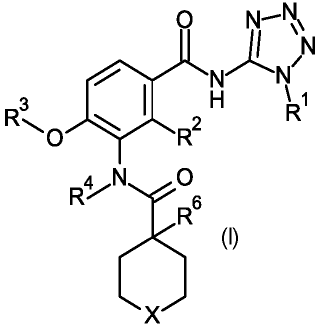
or an agronomically acceptable salt thereof,
wherein: -
X is selected from the group consisting of -O-, -S(O)ₚ- and -NR⁵;
R¹ is selected from the group consisting of C₁-C₄alkyl-, C₁-C₄haloalkyl-, C₁-C₄alkoxy-C₁-C₄alkyl- and C₁-C₄haloalkoxy-C₁-C₄alkyl-;
R² is selected from the group consisting of halogen, C₁-C₆alkyl-, C₁-C₃alkoxy-, C₁-C₆ haloalkyl-, C₁-C₃haloalkoxy- and -S(O)ₚC₁-C₆alkyl;
R³ is C₁-C₃haloalkyl;
R⁴ is selected from the group consisting of hydrogen, C₁-C₆alkyl-, C₁-C₆haloalkyl and C₃-C₆cycloalkyl;
R⁵ is selected from the group consisting of hydrogen, C₁-C₃alkyl- and C₁-C₃alkoxy-;
R⁶ is hydrogen or C₁-C₃alkyl; and
p is 0, 1 or 2.

2. A compound according to claim 1, wherein X is -O-.

3. A compound according to claim 1 or claim 2, wherein R¹ is selected from the group consisting of methyl, ethyl and n-propyl.

4. A compound according to any one of the previous claims, wherein R² is selected from the group consisting of methyl, Cl, -CF₃ and -SO₂ₘethyl.

5. A compound according to claim 3, wherein R² is Cl.

6. A compound according to any one of the previous claims, wherein R³ is -CF₃ or -CHF₂.

7. A compound according to any one of the previous claims, wherein R⁴ is hydrogen.

8. A compound according to any one of the previous clams, wherein R⁶ is hydrogen.

9. A herbicidal composition comprising a compound according to any one of the previous claims and an agriculturally acceptable formulation adjuvant.

10. A herbicidal composition according to claim 9, further comprising at least one additional pesticide.

11. A herbicidal composition according to claim 10, wherein the additional pesticide is a herbicide or herbicide safener.

12. A method of controlling weeds at a locus comprising application to the locus of a weed controlling amount of a composition according to any one of claims 9 to 11.

13. A method of making a compound of Formula (I) according to claim 1 comprising reacting a compound of Formula (II) 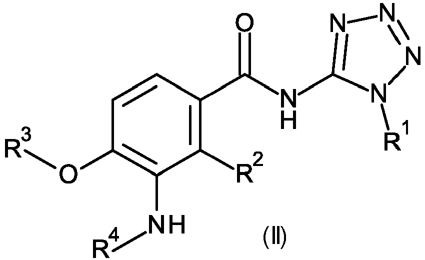 with a compound of Formula (III) 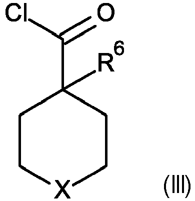 wherein X, R¹, R², R³, R⁴ and R⁶ are as defined in claim 1.

14. Use of a compound of Formula (I) as defined in claim 1 as a herbicide.

## Patentansprüche

1. Verbindung der Formel (I): 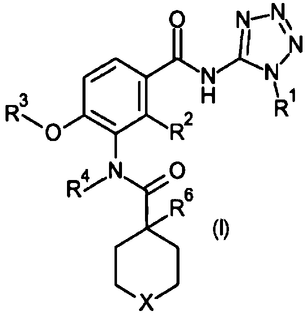
oder ein agronomisch unbedenkliches Salz davon,
wobei:
X aus der Gruppe bestehend aus -O-, -S(O)ₚ- und -NR⁵ ausgewählt ist;
R¹ aus der Gruppe bestehend aus C₁-C₄-Alkyl-, C₁-C₄-Halogenalkyl-, C₁-C₄-Alkoxy-C₁-C₄-alkyl- und C₁-C₄-Halogenalkoxy-C₁-C₄-alkyl- ausgewählt ist;
R² aus der Gruppe bestehend aus Halogen, C₁-C₆-Alkyl-, C₁-C₃-Alkoxy-, C₁-C₆-Halogenalkyl-, C₁-C₃-Halogenalkoxy- und -S(O)ₚ-C₁-C₆-Alkyl ausgewählt ist;
R³ für C₁-C₃-Halogenalkyl steht;
R⁴ aus der Gruppe bestehend aus Wasserstoff, C₁-C₆-Alkyl-, C₁-C₆-Halogenalkyl und C₃-C₆-Cycloalkyl ausgewählt ist;
R⁵ aus der Gruppe bestehend aus Wasserstoff, C₁-C₃-Alkyl- und C₁-C₃-Alkoxy- ausgewählt ist;
R⁶ für Wasserstoff oder C₁-C₃-Alkyl steht; und
p für 0, 1 oder 2 steht.

2. Verbindung nach Anspruch 1, wobei X für -O- steht.

3. Verbindung nach Anspruch 1 oder Anspruch 2, wobei R¹ aus der Gruppe bestehend aus Methyl, Ethyl und n-Propyl ausgewählt ist.

4. Verbindung nach einem der vorhergehenden Ansprüche, wobei R² aus der Gruppe bestehend aus Methyl, Cl, -CF₃ und -SO₂-Methyl ausgewählt ist.

5. Verbindung nach Anspruch 3, wobei R² für Cl steht.

6. Verbindung nach einem der vorhergehenden Ansprüche, wobei R³ für -CF₃ oder -CHF₂ steht.

7. Verbindung nach einem der vorhergehenden Ansprüche, wobei R⁴ für Wasserstoff steht.

8. Verbindung nach einem der vorhergehenden Ansprüche, wobei R⁶ für Wasserstoff steht.

9. Herbizide Zusammensetzung, umfassend eine Verbindung nach einem der vorhergehenden Ansprüche und ein landwirtschaftlich unbedenkliches Formulierungshilfsmittel.

10. Herbizide Zusammensetzung nach Anspruch 9, ferner umfassend mindestens ein zusätzliches Pestizid.

11. Herbizide Zusammensetzung nach Anspruch 10, wobei es sich bei dem zusätzlichen Pestizid um ein Herbizid oder einen Herbizid-Safener handelt.

12. Verfahren zur Bekämpfung von Unkräutern an einem Standort, bei dem man eine unkrautbekämpfende Menge einer Zusammensetzung nach einem der Ansprüche 9 bis 11 auf den Standort ausbringt.

13. Verfahren zur Herstellung einer Verbindung der Formel (I) nach Anspruch 1, umfassend das Umsetzen einer Verbindung der Formel (II) 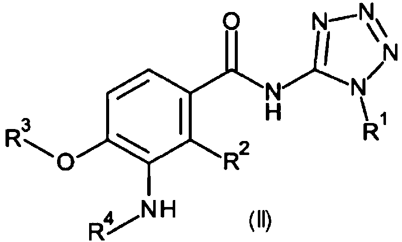 mit einer Verbindung der Formel (III) 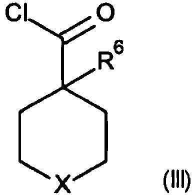 wobei X, R¹, R², R³, R⁴ und R⁶ wie in Anspruch 1 definiert sind.

14. Verwendung einer Verbindung der Formel (I) gemäß Anspruch 1 als ein Herbizid.

## Revendications

1. Composé de Formule (I) : 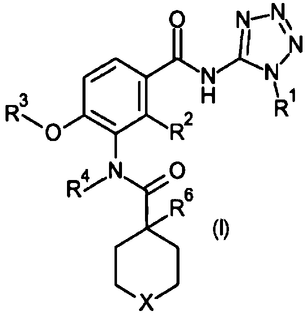
ou sel acceptable sur le plan agronomique correspondant,
X étant choisi dans le groupe constitué par -O-, -S(O)ₚ- et -NR⁵ ;
R¹ étant choisi dans le groupe constitué par C₁-C₄alkyle-, C₁-C₄halogénoalkyle-, C₁-C₄alcoxy-C₁-C₄alkyle- et C₁-C₄halogénoalcoxy-C₁-C₄alkyle- ;
R² étant choisi dans le groupe constitué par halogène, C₁-C₆alkyle-, C₁-C₃alcoxy-, C₁-C₆ halogénoalkyle-, C₁-C₃halogénoalcoxy- et -S(O)ₚC₁-Cₑalkyle ;
R³ étant C₁-C₃halogénoalkyle ;
R⁴ étant choisi dans le groupe constitué par hydrogène, C₁-C₆alkyle-, C₁-C₆halogénoalkyle et C₃-C₆cycloalkyle ;
R⁵ étant choisi dans le groupe constitué par hydrogène, C₁-C₃alkyle- et C₁-C₃alcoxy- ;
R⁶ étant hydrogène ou C₁-C₃alkyle ; et
p étant 0, 1 ou 2.

2. Composé selon la revendication 1, dans lequel X est -O-.

3. Composé selon la revendication 1 ou la revendication 2, dans lequel R¹ est choisi dans le groupe constitué par méthyle, éthyle et n-propyle.

4. Composé selon l'une quelconque des revendications précédentes, dans lequel R² est choisi dans le groupe constitué par méthyle, Cl, -CF₃ et -SO₂méthyle.

5. Composé selon la revendication 3, dans lequel R² est Cl.

6. Composé selon l'une quelconque des revendications précédentes, dans lequel R³ est -CF₃ ou -CHF₂.

7. Composé selon l'une quelconque des revendications précédentes, dans lequel R⁴ est hydrogène.

8. Composé selon l'une quelconque des revendications précédentes, dans lequel R⁶ est hydrogène.

9. Composition herbicide comprenant un composé selon l'une quelconque des revendications précédentes et un adjuvant de formulation acceptable sur le plan agricole.

10. Composition herbicide selon la revendication 9, comprenant en outre au moins un pesticide supplémentaire.

11. Composition herbicide selon la revendication 10, dans laquelle le pesticide supplémentaire est un herbicide ou un phytoprotecteur herbicide.

12. Procédé de lutte contre des mauvaises herbes au niveau d'un site comprenant une application sur le site d'une quantité de lutte contre des mauvaises herbes d'une composition selon l'une quelconque des revendications 9 à 11.

13. Procédé de préparation d'un composé de Formule (I) selon la revendication 1, comprenant la mise en réaction d'un composé de Formule (II) 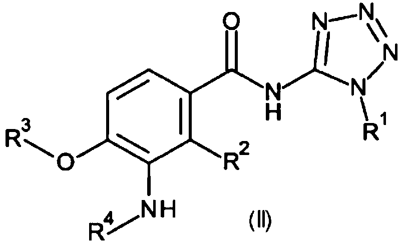 avec un composé de Formule (III) 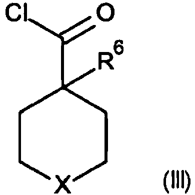 X, R¹, R², R³, R⁴ et R⁶ étant tels que définis dans la revendication 1.

14. Utilisation d'un composé de Formule (I) tel que défini dans la revendication 1, comme herbicide.
